# EUROPÄISCHE PATENTSCHRIFT

(11) **EP 2 528 642 B1**
(45) Veröffentlichungstag und Bekanntmachung des Hinweises auf die Patenterteilung: **22.11.2017**
(21) Anmeldenummer: 11701242.7
(22) Anmeldetag: 20.01.2011
(51) Int. Cl.: A61M 5/158, A61M 25/06

(54) **VERFAHREN ZUM HERSTELLEN EINES BAUTEILS MIT EINER KANÜLE, KANÜLE, BAUTEIL MIT EINER KANÜLE UND INSERTIONSKOPF**
METHOD FOR PRODUCING A COMPONENT HAVING A CANNULA, CANNULA, COMPONENT HAVING A CANNULA AND INSERTION HEAD
PROCÉDÉ DE FABRICATION D'UN ÉLÉMENT DOTÉ D'UNE CANULE, CANULE, ÉLÉMENT DOTÉ D'UNE CANULE ET TÊTE D'INSERTION

(30) Priorität: 25.01.2010 DE 102010005699
(43) Veröffentlichungstag der Anmeldung: 05.12.2012
(73) Patentinhaber: F. Hoffmann-La Roche AG, 4070 Basel (CH); Roche Diabetes Care GmbH, 68305 Mannheim (DE)
(72) Erfinder: ARGAUER, Herbert, 92712 Pirk (DE); HARTINGER, Josef, 92533 Wernberg-Köblitz (DE)
(74) Vertreter: Rentsch Partner AG
(86) Internationale Anmeldenummer: PCT/EP2011/050777
(87) Internationale Veröffentlichungsnummer: WO 2011/089193

(56) Entgegenhaltungen:
- EP-A1- 0 956 879
- EP-A1- 1 970 091
- US-A- 4 362 156

## Beschreibung

Die Erfindung betrifft zum einen ein Verfahren zum Herstellen eines Bauteils mit einer Kanüle zur subkutanen Verabreichung eines Wirkstoffs, bei welchem die Kanüle mit einem Kanülengehäuse verbunden wird.

Zum anderen betrifft die Erfindung eine Kanüle für ein Bauteil zur subkutanen Verabreichung eines Wirkstoffs, insbesondere an einem Applizierteil eines Insertionskopfes.

Des Weiteren betrifft die Erfindung ein Bauteil mit einer Kanüle zur subkutanen Verabreichung eines Wirkstoffs.

Außerdem betrifft die Erfindung einen Insertionskopf umfassend ein Bauteil mit einer Kanüle zur subkutanen Verabreichung eines Wirkstoffs.

Insbesondere ein gattungsgemäßer Insertionskopf, an welchem ein derartiges Bauteil mit einer Kanüle zur subkutanen Verabreichung eines Wirkstoffs eingesetzt wird, ist aus der europäischen Patentanmeldung EP 1 970 091 A1 bekannt. Der dort gezeigte Insertionskopf umfasst ein Kanülengehäuse in Gestalt eines Applizierteils, welches auf die Haut appliziert wird, wobei das Applizierteil eine Einstecheinrichtung - in Form einer Nadel - und eine flexible Einführeinrichtung - in Form einer flexiblen Kanüle - für einen Wirkstoff aufweist. Die Einstecheinrichtung stützt hierbei die flexible Einführeinrichtung beim Eindringen in die Haut und sie wird anschließend durch ein Septum hindurch aus der flexiblen Einführeinrichtung herausgezogen, sodass der Wirkstoff durch die flexible Einführeinrichtung hindurch in bzw. unter die Haut verabreicht werden kann. Das Septum befindet sich oberhalb der flexiblen Einführeinrichtung und es verschließt das Applizierteil nach dem Herausziehen der Einstecheinrichtung an der Stelle, an welcher sich diese zuvor befand.

Solche bekannten Applizierteile werden bisher fast immer derart hergestellt, dass in einem ersten Herstellungsschritt in eine flexible Kanüle ein Stabilisierungsdorn eingeführt wird, um die flexible Kanüle beim Herstellen eines dazugehörigen Kanülengehäuses besser verarbeiten zu können. Die so vorbereitete Baueinheit wird dann in ein vorproduziertes Kanülengehäuse eingebracht, um anschließend die flexible Kanüle und das Kanülengehäuse mittels eines thermischen Prozesses stabil miteinander verbinden zu können. Daran anschließend wird der Stabilisierungsdorn aus der flexiblen Kanüle heraus gezogen und durch das eigentliche Stechmittel ersetzt. Insbesondere beim Einstecken des Stechmittels in die flexible Kanüle des Kanülengehäuses kommt es oftmals zu Beschädigungen an der flexiblen Kanüle, was leider relativ hohe Ausschussraten zur Folge hat.

Es ist Aufgabe der Erfindung, die Herstellung und Haltbarkeit eines gattungsgemäßen Kanülengehäuses zu verbessern.

Die Aufgabe der Erfindung wird von einem Verfahren zum Herstellen eines Bauteils mit einer Kanüle zur subkutanen Verabreichung eines Wirkstoffs gelöst, bei welchem die Kanüle mit einem Kanülengehäuse verbunden wird, wobei sich das Verfahren dadurch auszeichnet, dass die lose Kanüle auf einem Stechmittel zu einer einsatzbereiten Kanülen-Stech-Einheit aufgefädelt und anschließend an die Kanüle ein Kanülengehäuse angespritzt wird.

Vorteilhafter Weise verringert sich hierdurch die Gefahr außerordentlich, dass die Kanüle durch das Stechmittel beschädigt wird, wodurch die Ausschussrate signifikant reduziert werden kann. Ebenso wird hierdurch die Haltbarkeit des Bauteils wesentlich verbessert, da vorteilhafter Weise durch die vorliegende Verfahrensführung Verletzungen an der Kanüle, welche zu einem Ausfall während des Einsatzes führen können, vermieden werden können.

Darüber hinaus können herkömmliche Herstellungsverfahren wesentlich vereinfacht werden, da vorliegend auf zusätzliche Herstellungsschritte und Bauteile, beispielsweise auf den Stabilisierungsdorn, verzichtet werden kann.

US 4362156 zeigt eine Infusionskanülenvorrichtung, bestehend aus einer Kanüleneinheit mit einer flexiblen Infusionskanüle und einer Einstechnadeleinheit mit einer starren Einstechnadel. Vor dem Einführen der Kanüle in die Vene eines Patienten sind die Einstechnadeleinheit und die Kanüleneinheit reversibel verriegelt, so dass die Nadel innerhalb der Kanüle dreh- und verschiebefest angeordnet ist. Nach dem Einführen in die Vene wird die Einstechnadeleinheit entriegelt, und die Nadel aus der Kanüle herausgezogen. Die Kanüleneinheit wird hergestellt, indem ein Kunststoffkörper auf eine schlauchförmige flexible Kanüle aufgespritzt wird. Die Einstechnadeleinheit wird hergestellt, indem ein Kunststoffkörper auf die starre Einstechnadel aufgespritzt wird. Anschliessend wird die Einstechnadel auf die Kanüle aufgefädelt, und die beiden Einheiten reversibel verriegelt.

EP 0956879 A1 stellt den nächstliegenden Stand der Technik dar und offenbart ein Verfahren gemäss dem Oberbegriff des Anspruchs 1 bzw. ein Bauteil mit Kanüle gemäss dem Oberbegriff des Anspruchs 6. Es zeigt einen Katheterkopf für die subkutane Verabreichung eines Wirkstoffs. Der Katheterkopf umfasst ein Kanülengehäuse mit einer flexiblen Kanüle, und einen reversibel lösbaren Anschluss für eine Wirkstoff-Zuleitung. Eine Einstechnadel ist in der Kanüle bündig angeordnet, und wird nach dem Platzieren des Katheters im Gewebe des Pattienten aus dem Katheter herausgezogen. Ein Septum dichtet die Kanüle nach dem Herausziehen der Einstechnadel nach aussen ab. Das Kanülengehäuse wird hergestellt, indem die Kanüle in das Spritzgusswerkzeug eingelegt und direkt umspritzt wird.

EP 1970091 A1 zeigt einen Insertionskopf zum Einbringen einer flexiblen Infusionskanüle mittels einer starren Einstechnadel in das Gewebe eines Patienten. Einstechnadel und Kanüle sind im Grundzustand des Insertionskopfs in einer Schutzposition innerhalb zweier zusammenschiebbarer Gehäusehälften angeordnet, und können durch Zusammenschieben der beiden Gehäusehälften ausgeklappt werden. Nach erfolgter Einführung der Kanüle ins Gewebe kann die Einstechnadel aus der Kanüle und dem diese tragenden Kanülengehäuse entfernt werden. Die Einstechnadel ist derart im Gehäuse des Insertionskopfes gehalten, dass sie direkt nach dem Austreten aus dem Kanülengehäuse durch Federkraft automatisch in eine Schutzposition im Gehäuse zurückgeschwenkt wird.

Das Bauteil kann als Applizierteil ausgestaltet sein, welches auf ein Gewebe, insbesondere auf einer Haut eines Patienten, appliziert wird, und dort zumindest für die Dauer einer Wirkstoffgabe verbleibt. Derartige Applizierteile sind aus dem Stand der Technik bereits gut bekannt und werden hier deshalb nicht weiter erläutert.

Der Begriff "Kanüle" beschreibt in vorliegendem Zusammenhang jegliche Einrichtung, mittels welcher ein Wirkstoff injiziert werden kann. Vorzugsweise handelt es sich hierbei um eine flexible Kanüle, also eine Softkanüle.

Es versteht sich, dass hinsichtlich der vorliegenden Kanüle verschiedenste Materialien, wie beispielsweise LLDPE, LDPE, HDPR, PTFE oder dergleichen zum Einsatz kommen können. Als besonders geeignet hat sich FEP 100 erwiesen, da es besonders gut in ein Gewebe, wie einem Hautgewebe, hinein gleiten kann. Vorzugsweise wird die Kanüle in der Farbe weiß ausgeführt.

Mit der Bezeichnung "Kanülengehäuse" wird eine Einrichtung beschrieben, welche die Kanüle aufnimmt bzw. an welcher die Kanüle angeschlossen ist. Ein solches Kanülengehäuse ist an sich bekannt und kann in einem Insertionskopf eingesetzt werden.

Vorzugsweise wird zum Herstellen des vorliegenden Kanülengehäuses das Material MABS verwendet, wobei auch andere Thermoplaste zum Einsatz kommen können, wie beispielsweise PC, PMMA, PP, PE oder dergleichen.

Insbesondere das Material MABS wird hinsichtlich des vorliegenden Herstellverfahrens idealerweise mit einer Temperatur zwischen 230°C und 260°C verarbeitet.

Eine außerordentlich gute Verarbeitung der Materialien beim Spritzgießen kann erzielt werden, wenn eine Werkzeugtemperatur zwischen 50°C und 80°C gewählt wird.

Liegt die Einspritzgeschwindigkeit des Materials bei 50 mm/s bis 150 mm/s, vorzugsweise bei 100 mm/s, kann die Verarbeitung nochmals verbessert werden.

Bei dem zu verabreichenden Wirkstoff handelt es sich idealerweise um medikamentöse Flüssigkeiten.

Als Stechmittel kommen jegliche Einrichtungen in Frage, mittels welchen die Kanüle unterstützend in ein Gewebe, speziell in die Haut, eingeführt werden kann. Insofern verbleibt dieses Stechmittel innerhalb der Kanüle, bis die Kanüle ordnungsgemäß in das Gewebe eingebracht wurde. Erst dann wird das Stechmittel in an sich bekannter Weise aus der Kanüle herausgezogen. Als mögliches Stechmittel kann vorzugsweise eine Nadel eingesetzt werden.

Der Begriff "Kanülen-Stech-Einheit" beschreibt im Sinne der Erfindung eine Baugruppe aus einer Kanüle und einem Stechmittel, wobei das Stechmittel zumindest teilweise innerhalb der Kanüle angeordnet ist.

Mittels dieser Kanülen-Stech-Einheit kann die Kanüle baulich sowie verfahrenstechnisch besonders einfach und stabil in ein Spritzgießwerkzeug eingelegt werden, um die Kanüle anschließend mit einem Material für das Kanülengehäuses zu umspritzen, sodass zumindest ein Ende der Kanüle fest in das Kanülengehäuse eingebettet ist.

Eine feste Verbindung zwischen Kanüle und Kanülengehäuse kann vorliegend sowohl durch Form- als auch durch Kraftschluss erzielt werden.

Kumulativ oder alternativ kann auch eine stoffschlüssige Verbindung der Materialien der Kanüle und des Kanülengehäuses vorgesehen werden.
Hinsichtlich eines weiteren Aspekts der Erfindung wird die Aufgabe ebenfalls von einem Verfahren zum Herstellen eines Bauteils mit einer Kanüle zur subkutanen Verabreichung eines Wirkstoffs gelöst, bei welchem die Kanüle mit einem Kanülengehäuse verbunden wird, wobei sich das Verfahren speziell dadurch auszeichnet, dass ein Ende der Kanüle zu einem von der Gestalt des übrigen Kanülenbereichs, insbesondere von einer zylindrischen Gestalt, abweichenden Endbereich geformt und anschließend an diesem Endbereich der Kanüle ein Kanülengehäuse angespritzt wird.

Um die Kanüle besonders betriebssicher mit dem Kanülengehäuse zu verbinden, wird vorteilhafter Weise ein Endbereich der Kanüle umgeformt, sodass er von der Gestalt der restlichen Kanüle abweicht. Vorteilhafter Weise kann hierdurch die Kanüle unmittelbar mit einer Verliersicherung ausgestattet werden, ohne dass hierfür weitere Bauteil erforderlich sind.

Hierdurch kann die Kanüle außergewöhnlich fest in dem Kanülengehäuse verankert werden. Dies ist besonders vorteilhaft, wenn der Wirkstoff unter Druck injiziert werden muss, wie dies etwa bei einer Insulingabe der Fall sein kann.

Insofern sollte die Verbindung zwischen der Kanüle und dem Kanülengehäuse Drücke von bis zu 4 bar problemlos verkraften können.

In einem sehr einfachen Fall reicht es bereits aus, wenn die Kanüle in einem ihrer Endbereiche einen anderen Durchmesser aufweist als in dem übrigen Kanülenbereich, sodass insbesondere im Endbereich der Kanüle eine Hinterschneidung ausbildet ist, die von dem Material des Kanülengehäuses umschlossen werden kann bzw. ist.

Unter diesem Aspekt wird die Aufgabe der Erfindung auch von einer Kanüle für ein Bauteil zur subkutanen Verabreichung eines Wirkstoffs, insbesondere an einem Applizierteil eines Insertionskopfes, gelöst, wobei die Kanüle einen Endbereich aufweist, der von der Gestalt des übrigen Kanülenbereichs abweicht.

Hierbei reicht es bereits aus, wenn der Durchmesser des Endbereichs größer ist als ein Durchmesser an einer anderen Stelle der Kanüle.

Ist ein Endbereich der Kanüle trichterförmig ausgestaltet, kann er bereits ausreichend gut eine Hinterschneidung ausbilden, welche förderlich von einem Material des Kanülengehäuses umschlossen werden kann. Insofern kann die vorliegende Kanüle unlösbar in das Kanülengehäuse eingebettet werden.

Eine weitere Steigerung der Verbindungsfestigkeit zwischen der Kanüle und dem Kanülengehäuse kann erreicht werden, wenn der trichterförmige Endbereich an seiner der Kanüle abgewandten Seite einen umlaufenden Wulst aufweist, der einen Kragen an dem trichterförmigen Endbereich ausgestaltet. Mit diesem Kragen kann die Kanüle nochmals verbessert gegen ein Herausrutschen aus dem Kanülengehäuse gesichert werden, da hierbei ein besonders guter Formschluss gewährleistet werden kann.

Ein derartiger Trichter kann auch als kreisförmiger, eckiger oder eine sonstige Querschnittsformen aufweisender umgefalteter Flansch ausgebildet sein, der vollständig mit einem Kunststoffmaterial des Kanülengehäuses umschlossen wird und hierdurch eine feste und dichte Verbindung generiert.

Sind der Endbereich der Kanüle und der übrige Kanülenbereich einstückig aus einem einzigen Material hergestellt, kann die vorliegende Kanüle sich vorteilhafter Weise durch einen besonders einfachen Aufbau auszeichnen.

Auch hinsichtlich der letzteren Verfahrensvariante ist es aus den bereits erläuterten Gründen vorteilhaft, wenn vor dem Anspritzen des Kanülengehäuses die Kanüle auf einem Stechmittel zu einer einsatzbereiten Kanülen-Stech-Einheit aufgefädelt wird, da sich hierdurch speziell die Herstellung der eingangs beschriebenen Applikationsteile wesentlich vereinfachen lässt.

Eine besonders bevorzugte Verfahrensvariante sieht vor, dass das Kanülengehäuse derart an die Kanüle angespritzt wird, dass das Stechmittel verschieblich das Kanülengehäuse durchdringt. Hierdurch kann insbesondere sichergestellt werden, dass das Stechmittel unmittelbar einsatzbereit verwendet werden kann.

Wird in einem weiteren Verfahrensschritt darüber hinaus nach dem Anspritzen des Kanülengehäuses endseitig auf das Stechmittel ein Septumelement aufgesteckt, welches in ein Lagerauge des Kanülengehäuses angeordnet wird, wobei insbesondere das Septumelement in das Lagerauge thermisch eingebördelt wird, kann eine Kammer, in welche das Stechmittel hineinragt, des Kanülengehäuses verfahrenstechnisch besonders einfach flüssigkeitsdicht verschlossen werden.

An dieser Stelle sei nochmals daraufhin gewiesen, dass es besonders vorteilhaft ist, dass das Septumelement erst nach dem Platzieren des Stechmittels in der Kanüle auf das Stechmittel aufgesteckt bzw. aufgefädelt wird.

Das Septumelement verschließt die Kammer des Kanülengehäuses selbst beim Entfernen des Stechmittels unmittelbar in an sich bekannter Weise.

Das vorliegende Herstellverfahren kann vorteilhaft weiterentwickelt werden, wenn in einem nächsten Verfahrensschritt anschließend endseitig an das Stechmittel eine Halteeinrichtung angebracht wird. Über eine solche Halteeinrichtung kann das Stechmittel mechanisch oder händisch besonders gut gehandhabt werden, sodass es bei der späteren Verwendung des Bauteils unproblematisch aus der Kanüle herausgezogen werden kann.

Die Halteeinrichtung kann an das Stechmittel geklebt werden. Vorzugsweise wird die Halteeinrichtung mittels eines UV-Klebers an das Stechmittel befestigt.

Vorteilhaft ist es weiter, wenn insbesondere die Spitze des Stechmittels zusammen mit der Außenseite der flexiblen Kanüle, also solange sich das Stechmittel noch in der flexiblen Kanüle befindet, mit einer Silikonschicht versehen wird, um ein besseres Hineingleiten insbesondere in ein Gewebe eines Körpers zu ermöglichen.

Des Weiteren wird die Aufgabe der Erfindung von einem Bauteil mit einer Kanüle zur subkutanen Verabreichung eines Wirkstoffs gelöst, wobei sich das Bauteil durch die hier beschriebene Kanüle auszeichnet.

Speziell durch eine derartige Kanüle kann das vorliegende Bauteil vorteilhaft hergestellt werden. Zudem verleiht die vorliegende Kanüle dem Bauteil eine besonders hohe Betriebssicherheit.

Darüber hinaus wird die Erfindung von einem Insertionskopf umfassend ein Bauteil mit einer Kanüle zur subkutanen Verabreichung eines Wirkstoffs gelöst, wobei sich der Insertionskopf durch das vorliegende Bauteil oder durch eine Aufnahme für ein derartiges Bauteil auszeichnet.

Weist der Insertionskopf ein solches Bauteil oder eine Aufnahme hierfür auf, kann auch dieser vorteilhaft bereitgestellt werden.

Die Erfindung kann somit vorrangig in einem Insertionskopf für medizinische oder pharmazeutische zur Anwendung kommen, der auf einem organischen Gewebe, wie menschlicher Haut, platzierbar ist. Hierfür weist der Insertionskopf das Stechmittel auf, das in das Gewebe eindringt, wenn der Insertionskopf auf dem Gewebe platziert wird oder ggf. auch erst nachdem der Insertionskopf auf dem Gewebe platziert wurde.

Häufig werden für eine Anwendung von Insulinverabreichungen mittels einer Insulinpumpe so genannte Transfersets verwendet, um eine Verbindung zwischen der Insulinpumpe und einem Körper aufzubauen. Ein so genanntes Headset, welches den Insertionskopf umfasst, ist in einem Schlauchsystem des Transfersets angeordnet und ist dafür vorgesehen, eine Verabreichung von Insulin mittels einer Nadel in den menschlichen Körper durchzuführen. Für die Anbringung derartiger Insertionsköpfe auf der Hautoberfläche des menschlichen Körpers sind beispielsweise Klebefolien vorgesehen, auf welchen die Insertionsköpfe angeordnet werden können und die eine klebende Verbindung mit der Hautoberfläche eingehen. Um das Insulin dem menschlichen Körper zuzuführen, ist nach erfolgtem Einstichvorgang die Nadel aus der ebenfalls in dem menschlichen Körper angeordneten Kanüle herauszuziehen, so dass anschließend über die Kanüle das Insulin zugeführt werden kann. Hierfür ist eine dauerhafte und druckfeste sowie flüssigkeitsdichte Verbindung zwischen dem Kunststoffbauteil, in diesem Fall in Form eines Kanülengehäuses, welches endseitig an dem einen Ende der Kanüle innerhalb des Insertionskopfes angeordnet ist, und dem Ende der Kanüle, insbesondere Kanüle, notwendig. Ansonsten würde nämlich insbesondere die Gefahr einer Falschdosierung bestehen, wodurch zu wenig Insulin in den Körper injiziert wird. Diese Gefahr ist vorliegend besonders gut ausgeschlossen.

Darüber hinaus ist mit dem erfindungsgemäßen Verfahren eine besonders exakte Bestimmung der Position der Kanüle gegenüber dem Kanülengehäuse während des Herstellungsprozesses möglich und vereinfacht somit auch eine Automatisierung des Verfahrens.

Im Stand der Technik wird mit der Nadel durch das Septumelement gestochen, welches die Kanüle verdeckt. Entsprechend groß ist die Wahrscheinlichkeit, dass die letztendliche Position von Nadel und Kanüle zueinander unpräzise ist. Diese Problematik wird noch durch die Tendenz verstärkt, dass die Nadel beim Durchstechen des Septumelements abdriftet. Dies trifft besonders bei unsymmetrisch geschliffenen Nadeln bzw. Nadelspitzen zu.

Vorliegend wird jedoch die Nadel bzw. das Stechmittel erfindungsgemäß in die noch nicht verbaute und noch nicht durch ein Septumelement verdeckte Kanüle auf- bzw. eingefädelt. Die Positionierung von Nadel und Kanüle zueinander ist entsprechend präziser. Der Einbau der Einheit von Nadel und Kanüle erfolgt vorteilhafter Weise erst nach diesem Auffädeln. Vorzugsweise wird das Septumelement erst danach von hinten über das Nadelende geschoben.

Es werden auch keine zusätzlichen Bauteile zur Fixierung der Kanüle in dem Kanülengehäuse benötigt. Insbesondere erübrigt sich hinsichtlich der Fixierung der Kanüle das Durchführen einer Ultraschallverbindung mittels einer Ultraschallsonotrode oder das Durchführen einer anderen Verbindungsmethode, wie beispielsweise Kleben, um die Kanüle noch vor dem Platzieren der Nadel bzw. des Septumelements in dem Kanülengehäuse zu fixieren. Vorliegend kann auf einen solchen zusätzlichen Fertigungsschritt verzichtet werden, da die Kanüle samt der Nadel mit dem Kanülengehäuse umspritzt wird. Insofern kann gegenüber den herkömmlichen Herstellverfahren zusätzlich sowohl eine Zeit- als auch Kostenersparnis erzielt werden.

Durch die bereits anfängliche Anordnung des Stechmittels innerhalb der Kanüle, welche die erfindungswesentliche Endausbildung im oberen Bereich aufweist, ist nach erfolgtem Umspritzen des Endes der Kanüle mit dem Kunststoffmaterial des Kanülengehäuses keine nachträgliche Einführung eines Stechmittels in die Kanüle notwendig. Hierdurch werden Beschädigungen insbesondere des oberen Kanülenendes oder auch der benachbarten Anteile des Kanülengehäuses verhindert. Es erübrigt sich deshalb ein Einfädeln der Nadel in eine Kanüle. Durch das Vermeiden eines solchen Einfädelschrittes, wie oben erwähnt, kann eine schnelle und automatisierte Produktion bzw. Herstellung des Kanülengehäuses zusammen mit der Kanüle für Insertionsköpfe erfindungsgemäß durchgeführt werden.

Zudem ist eine derartige Herstellung kostengünstig, da sie zum einen schnell durchführbar ist und zum anderen das Vorliegen und Einbringen eines Dorns für die Anordnung der Kanüle innerhalb des Kanülengehäuses, wie es bisher beim dem Stand der Technik üblich war, nicht mehr notwendig ist. Vielmehr wird nun anstelle des Dorns das Stechmittel als Führungselement bei der Herstellung verwendet.

Insofern wird die Aufgabe der Erfindung auch von einer Verwendung eines Stechmittels als Führungsdorn für eine Kanüle zur subkutanen Verabreichung eines Wirkstoffs gelöst.

Weitere Vorteile, Ziele und Eigenschaften vorliegender Erfindung werden anhand nachfolgender Beschreibung anliegender Zeichnung erläutert, in welcher beispielhaft ein Herstellverfahren und ein entsprechender Aufbau eines Bauteils zur subkutanen Verabreichung eines Wirkstoffs und eine Insertionsvorrichtung hierzu dargestellt ist.

Es zeigen
- Figur 1A: schematisch einen ersten Herstellungsschritt eines Bauteils zur subkutanen Verabreichung eines Wirkstoffs mit einer Kanüle und einem Kanülengehäuse;
- Figur 1B: schematisch einen weiteren Herstellungsschritt eines Bauteils;
- Figur 1C: schematisch einen nächsten Herstellungsschritt eines Bauteils;
- Figur 1D: schematisch einen weiteren Herstellungsschritt eines Bauteils;
- Figur 2: schematisch eine Explosionsansicht des fertigen Bauteils;
- Figur 3: schematisch eine Detailansicht des fertigen Bauteils;
- Figur 4: schematisch eine alternative und bevorzugte Ausgestaltung einer Kanüle;
- Figur 5: schematisch eine Detailansicht der bevorzugten Kanüle aus der Figur 4 an einem Kanülengehäuse; und
- Figur 6: schematisch eine teilweise geschnittene Ansicht einer an sich bekannten Insertionsvorrichtung mit einem erfindungsgemäßen Bauteil zur subkutanen Verabreichung eines Wirkstoffs.

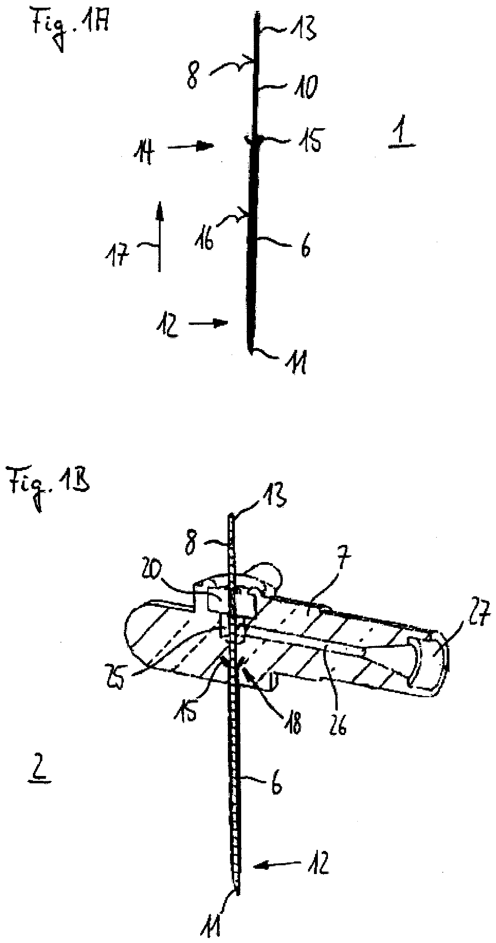

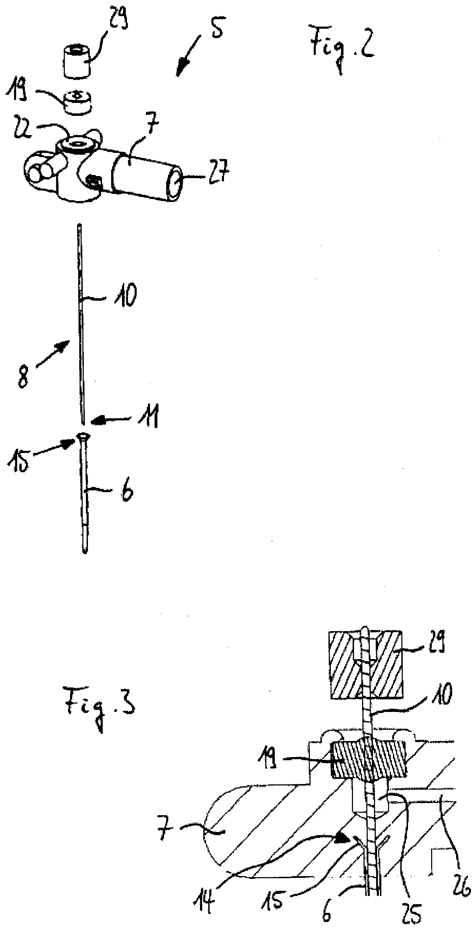

In den Figuren 1A bis 1D werden die wesentlichen Herstellungsschritte 1 bis 4 zur Produktion eines Bauteils 5 (siehe insbesondere Figur 2) mit einer flexiblen Kanüle 6 und einem dazugehörigen Kanülengehäuse 7 (siehe ab Figur 1B) erläutert.

Bei dem ersten Herstellungsschritt 1 gemäß der Figur 1A wird die flexible Kanüle 6 auf einem Stechmittel 8 zu einer Kanülen-Stech-Einheit 9 aufgefädelt, wobei das Stechmittel 8 in diesem Ausführungsbeispiel eine Nadel 10 ist. Die flexible Kanüle 6 ist hierbei als Softkanüle aus einem Polymermaterial ausgeführt.

Hierbei steht einerseits die Spitze 11 des Stechmittels 8 vorderseitig über einen ersten Endbereich 12 der flexiblen Kanüle 6 hervor. Andererseits steht ein Ende 13 des Stechmittels 8 rückseitig über einen zweiten Endbereich 14 der flexiblen Kanüle 6 über.

Der zweite Endbereich 14 ist in diesem Ausführungsbeispiel zu einem Trichter 15 geformt. Insofern weicht die Gestalt des zweiten Endbereichs 14 von der zylindrischen Gestalt des übrigen Kanülenbereichs 16 ab.

Zudem ermöglicht eine derartige trichterförmige Ausbildung der flexiblen Kanüle 6 vorteilhaft das ungewollte Heraustreten von Flüssigkeiten, wie Insulin, zwischen einer Kanülenaußenwand und dem Kanülengehäuse 7, da die Trichterform nicht nur eine vergrößerte Einlassöffnung für das anschließend durch die Kanüle 6 hindurch fließende Insulin zur Verfügung stellt, sondern auch eine in sich feste Verbindung mit dem den Trichter 15 umgebenden Kunststoff des Kanülengehäuses 7 ermöglicht. Dies hat vorteilhaft zur Folge, dass die Verbindung zwischen dem Kanülengehäuse 7 und der flexiblen Kanüle 6 problemlos Drücke von bis zu 4 bar oder mehr bei Zufluss von Insulin dichtend aushalten kann.

Die flexible Kanüle 6 wurde hierbei trichterseitig in Richtung des Pfeils 17 von der vorderseitigen Spitze 11 auf das Stechmittel 8 aufgefädelt.

Bei dem zweiten Herstellungsschritt 2 gemäß der Figur 1B wird die flexible Kanüle 6 im Bereich des Trichters 15, also im zweiten Endbereich 14 der flexiblen Kanüle 6, mit einem weiteren Polymermaterial umspritzt, sodass nunmehr an der flexible Kanüle 6 das Kanülengehäuse 7 flüssigkeits- und druckdicht angespritzt ist.

Gut zu erkennen ist, dass der Trichter 15 eine Hinterschneidung 18 ausgestaltet, welche von dem weiteren Polymermaterial des Kanülengehäuses 7 umschlossen werden kann, sodass die flexible Kanüle 6 besonders sicher gegen ein Herausrutschen aus dem Kanülengehäuse 7 gesichert ist.

Für diesen speziellen Spritzvorgang wurde die Kanülen-Stech-Einheit 9 in ein geeignetes Spritzgießwerkzeug (hier nicht gezeigt) eingelegt. Das Spritzgießwerkzeug ist hierbei derart konstruiert, dass das Stechmittel 8 nicht fest mit dem Kanülengehäuse 7 in Verbindung steht, sodass das Stechmittel 8 das Kanülengehäuse 7 verschieblich durchdringt.

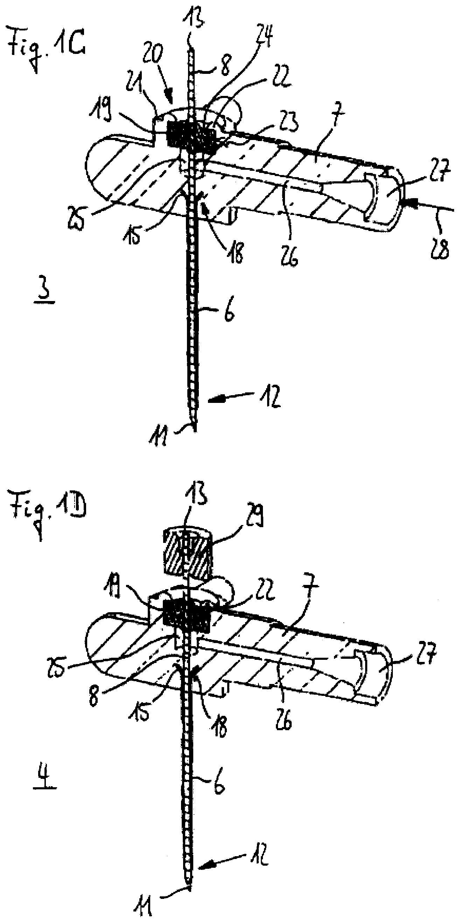

Bei dem dritten Herstellungsschritt 3 gemäß der Figur 1C wird ein scheibenförmiges Septumelement 19 endseitig - also über das Ende 13 des Stechmittels 8 - auf das Stechmittel 8 aufgesteckt und in ein Lagerauge 20 (siehe auch Figur 1B) des Kanülengehäuses 7 hinein geschoben.

Das Septumelement 19 wird anschließend mittels einer thermischen Behandlung des Kanülengehäusebereichs 21 mittels eines randseitigen Wulstes 22 eingebördelt, sodass das Septumelement 4 an der Seitenfläche 23 und der Oberseite 24 von dem randseitigen Wulst 22 fest eingeschlossen ist. Dies ergibt insbesondere eine septisch dichte Wirkung gegenüber einer Kammer 25, in welche ein flüssiger Wirkstoff, wie beispielsweise Insulin, über einen röhrenförmigen Kanal 26 des Kanülengehäuses 7 zugeleitet wird, wenn das Stechmittel 8 bei einer Anwendung entfernt wurde. Aus der Kammer 25 gelangt der flüssige Wirkstoff in die flexible Kanüle 6 und von dort aus etwa in einen menschlichen Körper.

Der flüssige Wirkstoff wird dem röhrenförmigen Kanal 26 hierzu mittels eines hier nicht näher dargestellten Katheters zugeleitet, wobei der Katheter an einer Anschlussstelle 27 an das Kanülengehäuse 7 angeschlossen wird. Der flüssige Wirkstoff gelangt somit gemäß der Zuführrichtung 28 in das Kanülengehäuse 7 hinein.

Bei dem vierten Herstellungsschritt 4 gemäß der Figur 1D wird noch eine Halteeinrichtung 29 rückseitig an das Stechmittel 8, also an das Ende 13 des Stechmittels 8, angebracht.

Mittels der Halteeinrichtung 29 kann das Stechmittel 8 während einer Verwendung aus der Kanüle 6 heraus gezogen werden, und hierbei insbesondere automatisiert auf einfache Weise schnell entfernt werden.

Das mittels des erfindungsgemäßen Verfahrens hergestellte Bauteil 5 kann hinsichtlich seiner wesentlichen Komponenten übersichtlich in der Figur 2 überblickt werden. Neben den allgemeinen Grundformen der einzelnen Komponenten kann auch besonders gut erkannt werden, dass die flexible Kanüle 6 mittels des Trichters 15 besonders betriebssicher auf das Stechmittel 8 aufgefädelt werden kann.

Insofern kann der Trichter 15 eine sehr vorteilhafte Einfädeleinrichtung an der flexiblen Kanüle 6 darstellen, sodass das Stechmittel 8 zielgerichtet und zentriert in die flexible Kanüle 6 eingeführt werden kann. Somit ist baulich eine besonders einfache Zentriereinrichtung geschaffen. Es versteht sich, dass eine derartige vorteilhafte Zentriereinrichtung bzw. Einfädeleinrichtung nicht auf die hier beispielhaft gezeigte Trichterform beschränkt ist.

Nach der Darstellung der Figur 3 ist das zusammengesetzte und einsatzbereite Bauteil 5 im Bereich der flexiblen Kanüle 6 und des Stechmittels 8 teilweise nochmals vergrößert dargestellt.

Der trichterförmige zweite Endbereich 14 der flexiblen Kanüle 6 gleicht in etwa einem innen hohl ausgebildeten Kegelstumpf. Diese Trichterform ist besonders einfach in einem Umformverfahren, insbesondere aber auch urformend in einem Spritzgießverfahren, herstellbar. Es könnte allerdings auf Grund dieser einfachen Trichterform nach sehr langer Dauer möglich sein, dass sich dennoch die flexible Kanüle 6 aus ihrem Sitz am Kanülengehäuse löst.

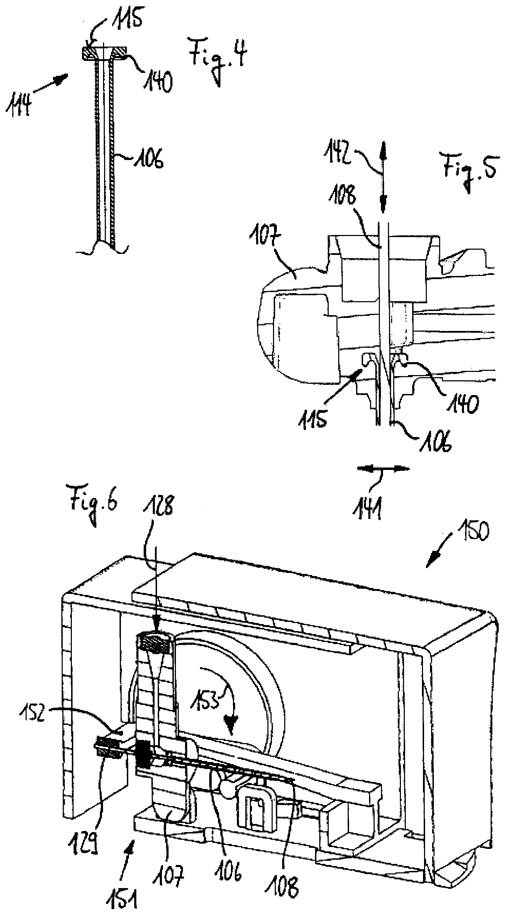

Um selbst dieses geringe Risiko besser auszuschließen zu können, kann die Trichterform auch anders gestaltet sein, wie dies mit einem anderen Ausführungsbeispiel einer alternativen und bevorzugten Softkanüle 106 nach den Figuren 4 und 5 beispielhaft gezeigt ist.

Durch den wulstigen Bereich 140 an einem Endbereich 114 der Softkanüle 106 kann der dortige Trichter 115 wesentlich besser von einem Material eines Kanülengehäuses 107 umspritzt werden, sodass die Softkanüle 106 innerhalb des Materials des Kanülengehäuses 107 nochmals verbessert arretiert ist. Insofern ist ein Loslösen der Softkanüle 106 aus dem Kanülengehäuse 107 hierbei eher vollständig ausgeschlossen.

Hierbei ist das Stechmittel 108 (siehe Figur 5) in radialer Richtung 141 im Wesentlichen von der Softkanüle 106 fixiert. In axialer Richtung 142 ist das Stechmittel 108 hingegen nahezu reibungsfrei innerhalb der Softkanüle 108 verschieblich gelagert.

Gemäß der Darstellung nach der Figur 6 ist eine an sich bekannte Insertionsvorrichtung 150 mit einem Insertionskopf 151 in einem inaktiven Zustand umfassend das Kanülengehäuse 107 und der darin angeordneten Softkanüle 108 zu sehen.

Eine gattungsgemäße Halteeinrichtung 129 ist hierbei als ein Gehäuseelement 152 der Insertionsvorrichtung 150 ausgebildet.

Nach einem Schwenkvorgang gemäß der Schwenkrichtung 153, einem Applizieren des Insertionskopfes 151 auf einem Gewebe und einem Entfernen des Stechmittels 108 aus der Softkanüle 106 kann Insulin in Zuführrichtung 128 in das Kanülengehäuse 107 geleitet werden.

Die Insertionsvorrichtung 150 weist die vorstehend beschriebenen Komponenten auf und ermöglicht ein Schwenken des Kanülengehäuses 107 um 90°., um hierdurch ein Einstechen in das Gewebe, insbesondere in eine Hautoberfläche, zu ermöglichen.

Die genaue Funktionsweise einer derartigen Insertionsvorrichtung 150 ist aus dem Stand der Technik, beispielsweise aus der eingangs erwähnten europäischen Patentanmeldung EP 1 970 091 A1, hinreichend bekannt.

Sämtliche in den Anmeldungsunterlagen offenbarten Merkmale werden als erfindungswesentlich beansprucht, sofern sie einzeln oder in Kombination miteinander gegenüber dem Stand der Technik neu sind.

### Bezugszeichenliste

- 1: erster Herstellungsschritt
- 2: zweiter Herstellungsschritt
- 3: dritter Herstellungsschritt
- 4: vierter Herstellungsschritt
- 5: Bauteil
- 6: flexible Kanüle
- 7: Kanülengehäuse
- 8: Stechmittel
- 9: Kanülen-Stech-Einheit
- 10: Nadel
- 11: Spitze
- 12: erster Endbereich
- 13: Ende
- 14: zweiter Endbereich
- 15: Trichter
- 16: übriger Kanülenbereich
- 17: Pfeil
- 18: Hinterschneidung
- 19: scheibenförmiges Septumelement
- 20: Lagerauge
- 21: Kanülengehäusebereich
- 22: randseitiger Wulst
- 23: Seitenfläche
- 24: Oberseite
- 25: Kammer
- 26: röhrenförmiger Kanal
- 27: Anschlussstelle
- 28: Zuführrichtung
- 29: Halteeinrichtung

- 106: Softkanüle
- 107: Kanülengehäuse
- 108: Stechmittel
- 115: Trichter
- 128: Zuführrichtung
- 140: wulstiger Bereich
- 141: radiale Richtung
- 142: axiale Richtung
- 150: Insertionsvorrichtung
- 151: Insertionskopf
- 152: Gehäuseelement
- 153: Schwenkrichtung

## Patentansprüche

1. Verfahren zum Herstellen eines Bauteils (5) mit einer Kanüle (6, 106) zur subkutanen Verabreichung eines Wirkstoffs, bei welchem die Kanüle (6, 106) mit einem Kanülengehäuse (7, 107) verbunden wird, **dadurch gekennzeichnet, dass** die lose Kanüle (6, 106) auf einem Stechmittel (8, 108) zu einer einsatzbereiten Kanülen-Stech-Einheit (9) aufgefädelt und anschliessend an die Kanüle (6, 106) ein Kanülengehäuse (7, 107) angespritzt wird.

2. Verfahren nach Anspruch 1, **dadurch gekennzeichnet, dass** ein Ende der Kanüle (6, 106) zu einem von der Gestalt des übrigen Kanülenbereichs (16) abweichenden Endbereich (14, 114) geformt und anschliessend an diesem Endbereich (14, 114) der Kanüle (6, 106) ein Kanülengehäuse (7, 107) angespritzt wird.

3. Verfahren nach einem der vorangegangenen Ansprüche, **dadurch gekennzeichnet, dass** das Kanülengehäuse (7, 107) derart an die Kanüle (6, 106) angespritzt wird, dass ein Stechmittel (8, 108) verschieblich das Kanülengehäuse (7, 107) durchdringt.

4. Verfahren nach einem der vorangegangenen Ansprüche, **dadurch gekennzeichnet, dass** nach dem Anspritzen des Kanülengehäuses (7, 107) endseitig auf ein Stechmittel (8, 108) ein Septumelement (19) aufgesteckt und in ein Lagerauge (20) des Kanülengehäuses (7, 107) angeordnet wird, wobei insbesondere das Septumelement (19) in das Lagerauge (20) thermisch eingebördelt wird.

5. Verfahren nach einem der vorangegangenen Ansprüche, **dadurch gekennzeichnet, dass** anschliessend endseitig (13) an ein Stechmittel (8, 108) eine Halteeinrichtung (29, 129) angebracht wird.

6. Bauteil (5) mit einer Kanüle (6, 106) zur subkutanen Verabreichung eines Wirkstoffs, wobei die Kanüle einen Endbereich (14, 114) aufweist, der von der Gestalt des übrigen Kanülenbereichs (16) abweicht; mit einem Kanülengehäuse (7, 107), mit welchem die Kanüle formschlüssig verbunden ist; und mit einem Stechmittel (8, 108), auf welches die Kanüle zu einer einsatzbereiten Kanülen-Stech-Einheit (9) aufgefädelt ist,
**dadurch gekennzeichnet, dass** das Bauteil mit einem Verfahren nach einem der Ansprüche 1 bis 5 hergestellt ist, und dass das Kanülengehäuse derart an die Kanüle angespritzt ist, dass der Endbereich der Kanüle vollständig von einem Kunststoffmaterial des Kanülengehäuses umschlossen ist, wobei das Stechmittel das Kanülengehäuse verschieblich durchdringt, so dass nach dem Entfernen des Stechmittels bei der Anwendung im Kanülengehäuse ein röhrenförmiger Kanal verbleibt, welcher die Kanüle mit einer Kammer (25) des Kanülengehäuses verbindet, welche mit einer Anschlussstelle (27) des Kanülengehäuses für die Zuleitung (28) des flüssigen Wirkstoffes fluidisch verbunden (26) ist.

7. Bauteil nach Anspruch 6, **dadurch gekennzeichnet, dass** der Endbereich (14, 114) der Kanüle (6, 106) und der übrige Kanülenbereich einstückig aus einem einzigen Material hergestellt sind.

8. Bauteil nach Anspruch 6 oder 7, **dadurch gekennzeichnet, dass** die Kanüle (6, 106) als flexible Kanüle ausgestaltet ist.

9. Bauteil nach einem der Ansprüche 6 bis 8, **dadurch gekennzeichnet, dass** der Endbereich (14, 114) der Kanüle (6, 106) als Trichter (15, 115) ausgestaltet ist.

10. Bauteil nach Anspruch 9, **dadurch gekennzeichnet, dass** der trichterförmige Endbereich (114) der Kanüle (106) einen Kragen in Form eines umlaufenden Wulstes (140) aufweist.

11. Insertionskopf (151) mit einem Bauteil (5) nach einem der Ansprüche 6 bis 10, wobei das Bauteil (5) gemäss einem Verfahren nach einem der Ansprüche 1 bis 5 hergestellt ist.

## Claims

1. A method for producing a component (5) with a cannula (6, 106) for the subcutaneous administration of an active substance, wherein the cannula (6, 106) is connected to a cannula housing (7, 107), **characterised in that** the loose cannula (6, 106) is threaded onto a piercing means (8, 108) to form a ready-to-use cannula piercing unit (9) and a cannula housing (7, 107) is subsequently moulded onto the cannula (6, 106).

2. The method according to claim 1, **characterised in that** one end of the cannula (6, 106) is shaped to form an end region (14, 114) which is different from the shape of the remaining cannula region (16), and subsequently a cannula housing (7, 107) is moulded onto this end region (14, 114) of the cannula (6, 106).

3. The method according to one of the preceding claims, **characterised in that** the cannula housing (7, 107) is moulded onto the cannula (6, 106) such that a piercing means (8, 108) movably penetrates the cannula housing (7, 107) .

4. The method according to one of the preceding claims, **characterised in that** after the cannula housing (7, 107) has been moulded-on, a septum element (19) is fitted onto the end of a piercing means (8, 108) and arranged in a bearing eye (20) of the cannula housing (7, 107), wherein in particular the septum element (19) is thermally crimped into it.

5. The method according to one of the preceding claims, **characterised in that** subsequently a holding device (29, 129) is attached to an end (13) of a piercing means (8, 108).

6. A component (5) with a cannula (6, 106) for the subcutaneous administration of an active substance, wherein the cannula has an end region (14, 114), which is different from the shape of the remaining cannula region (16); with a cannula housing (7, 107), to which the cannula is connected in a form-locked manner; and with a piercing means (8, 108), onto which the cannula is threaded to form a ready-to-use cannula piercing unit (9),
**characterised in that** the component is produced by a method according to one of claims 1 to 5, and **in that** the cannula housing is moulded onto the cannula such that the end region of the cannula is completely enclosed by a plastic material of the cannula housing, wherein the piercing means penetrates the cannula housing in a movable manner, so that after removing the piercing means during the application a tubular channel remains in the cannula housing, which connects the cannula with a chamber (25) of the cannula housing, which chamber is fluidically connected to a connecting point (27) of the cannula housing for the feeding in (28) of the liquid active substance.

7. The component according to claim 6, **characterised in that** the end region (14, 114) of the cannula (6, 106) and the remaining cannula region are produced in one piece from a single material.

8. The component according to claim 6 or 7, **characterised in that** the cannula (6, 106) is formed as a flexible cannula.

9. The component according to one of claims 6 to 8, **characterised in that** the end region (14, 114) of the cannula (6, 106) is shaped as a funnel (15, 115).

10. The component according to claim 9, **characterised in that** the funnel-shaped end region (114) of the cannula (106) has a collar in the shape of an encircling bead (140).

11. An insertion head (151) with a component (5) according to one of claims 6 to 10, wherein the component (5) is produced according to a method as specified in one of claims 1 to 5.

## Revendications

1. Procédé de fabrication d'un élément (5) doté d'une canule (6,106) pour l'administration sous-cutanée d'une substance active, pour lequel la canule (6,106) est reliée à un boîtier de canule (7,107), **caractérisé en ce que** la canule (6, 106) mobile est enfilée sur un moyen de perçage (8,108) en une unité canule-perçage (9) prête à l'emploi et un boîtier de canule (7,107) est ensuite moulé par injection sur la canule (6,106).

2. Procédé selon la revendication 1, **caractérisé en ce qu'**une extrémité de la canule (6,106) est formée en une zone d'extrémité (14,114) dérivant de la structure de la zone de canule restante (16) et un boîtier de canule (7,107) est ensuite moulé par injection sur cette zone d'extrémité (14,114) de la canule (6,106).

3. Procédé selon l'une quelconque des revendications précédentes, **caractérisé en ce que** le boîtier de canule (7,107) est moulé par injection sur la canule (6,106) de telle sorte qu'un moyen de perçage (8,108) traverse de façon mobile le boîtier de canule (7,107).

4. Procédé selon l'une quelconque des revendications précédentes, **caractérisé en ce qu'**après le moulage par injection du boîtier de canule (7,107), un élément septal (19) est enfoncé en extrémité sur un moyen de perçage (8,108) et est disposé dans un oeillet de support (20) du boîtier de canule (7,107), l'élément septal (19) étant en particulier thermiquement serti de bord dans l'oeillet de support (20).

5. Procédé selon l'une quelconque des revendications précédentes, **caractérisé en ce qu'**un dispositif de maintien (29,129) est monté ensuite en extrémité (13) sur un moyen de perçage (8,108).

6. Elément (5) avec une canule (6,106) pour l'administration sous-cutanée d'une substance active, la canule comportant une zone d'extrémité (14,114), qui dérive de la structure de la zone de canule restante (16), avec un boîtier de canule (7,107), avec lequel la canule est reliée par conformité de forme et avec un moyen de perçage (8,108) sur lequel la canule est enfilée à une unité canule-perçage prête à l'emploi (9), **caractérisé en ce que** l'élément est fabriqué avec un procédé selon l'une quelconque des revendications 1 à 5 et **en ce que** le boîtier de canule est moulé par injection de telle sorte que la zone d'extrémité de la canule est complètement englobée d'une matière plastique du boîtier de canule, le moyen de perçage traversant le boîtier de canule de façon mobile de telle sorte qu'après enlèvement du moyen de perçage lors de l'emploi, il reste un canal de forme tubulaire dans le boîtier de canule, lequel relie la canule à une chambre (25) du boîtier de canule, laquelle est reliée de manière fluide (26) à un point de raccord (27) du boîtier de canule pour l'alimentation (28) de la substance active liquide.

7. Elément selon la revendication 6, **caractérisé en ce que** la zone d'extrémité (14,114) de la canule (6,106) et la zone de canule restante sont fabriquées en une seule pièce à partir d'un matériau unique.

8. Elément selon la revendication 6 ou 7, **caractérisé en ce que** la canule (6,106) est configurée en tant que canule souple.

9. Elément selon l'une quelconque des revendications 6 à 8, **caractérisé en ce que** la zone d'extrémité (14,114) de la canule (6,106) est constituée comme entonnoir (15,115).

10. Elément selon la revendication 9, **caractérisé en ce que** la zone d'extrémité en forme d'entonnoir (114) de la canule (106) comporte un col sous la forme d'un bourrelet périphérique (140).

11. Tête d'insertion (151) avec un élément (5) selon l'une quelconque des revendications 6 à 10, l'élément (5) étant fabriqué conformément à un procédé selon l'une quelconque des revendications 1 à 5.
